# EUROPEAN PATENT SPECIFICATION

(11) **EP 4 420 633 B1**
(45) Date of publication and mention of the grant of the patent: **23.07.2025**
(21) Application number: 23810933.4
(22) Date of filing: 18.05.2023
(51) Int. Cl.: A61F 2/00

(54) **AUXILIARY PRESSURE APPLYING DEVICE FOR STRESS URINARY INCONTINENCE**
HILFSDRUCKVORRICHTUNG FÜR STRESSBEDINGTE HARNINKONTINENZ
DISPOSITIF D'APPLICATION DE PRESSION AUXILIAIRE POUR INCONTINENCE URINAIRE À L'EFFORT

(30) Priority: 23.05.2022 CN 202210564541
(43) Date of publication of application: 28.08.2024
(73) Proprietor: Jiangsu Tech-Bio-Med Medical Equipment Co., Ltd., Changzhou, Jiangsu 213100 (CN)
(72) Inventor: GUO, Yanlin, hangzhou, Jiangsu 213100 (CN); ZHANG, Wei, hangzhou, Jiangsu 213100 (CN)
(74) Representative: Bayramoglu et al.
(86) International application number: PCT/CN2023/094930
(87) International publication number: WO 2023/226860

(56) References cited:
- EP-B1- 2 214 587
- WO-A1-2008/079271
- WO-A2-2018/078635
- CN-A- 101 001 584
- CN-A- 103 391 761
- CN-A- 114 848 228
- US-A1- 2009 203 959
- US-A1- 2009 203 959
- US-A1- 2017 014 217
- US-B1- 6 676 594

## Description

### Technical Field

The present invention relates to the technical field of medical devices, and in particular to an auxiliary pressure device for stress urinary incontinence.

### Background

Stress urinary incontinence, or SUI for short, is mainly caused by the relaxation of the pelvic floor supporting tissue that results in a decrease in the pressure in the urethra. Usually, when the patient exerts force on the abdomen, such as sneezing, coughing, laughing, or walking quickly, the intra-abdominal pressure will increase, causing involuntary urine leakage from the external urethra orifice. SUI can be unpleasant and embarrassing for women in life, and the pessary, which has been most commonly used for SUI, has become a feasible non-surgical option for treating SUI.

However, when the existing pessary is placed in the vagina for a long time, it will cause discomfort to the user, and there is a risk of falling off during use. Therefore, it still requires significant medical intervention during the use of the pessary.

In view of the above problems, how to obtain a comfortable and convenient vaginal implant to alleviate the SUI-related problems currently faced by women has become an urgent problem to be solved.

WO 2008/079271 A1 discloses a compactable vaginal pessary for managing pelvic organ prolapse. The compactable pessary easily expands once inside a patient's vagina, remaining firmly anchored in the vaginal cavity during movement, thereby effectively countering the effects of any prolapsing organ. EP 2 214 587 B1 discloses an apparatus for treating urinary incontinence, comprising: (a) a support section adapted for providing urethral support; (b) an anchoring section adapted for resisting movement of the apparatus; (c) a normally open expansion mechanism adapted to urge the support radially outwards; and (d) a conversion mechanism adapted to sharply and selectively reduce an outward urging of said support. WO 2018/078635 A2 discloses a fecal incontinence device for insertion into a vagina, comprising: a plurality of shell segments, which together form an enclosed cylinder with rounded ends in a collapsed state of the device, wherein one of the cylindrical shells is disposed in the device facing a posterior wall of the vagina when the device is inserted into the vagina; a pressure generating structure attached to and abutting the posterior-facing cylindrical shell; and, a state-changing mechanism configured to reversibly transition the device from the collapsed state to an expanded state. US 2009/203959 A1 discloses a female urinary incontinence-inhibiting device comprising a flexible torus including at least one slot configured to, under compression, flexibly reduce the outer diameter of the flexible torus, the flexible torus having a diameter configured to inhibit female urinary incontinence when placed in a vagina and a stabilizing element, coupled to the flexible torus, the stabilizing element having a size and position configured to stabilize the flexible torus within the vagina.

### Summary

The present invention provides an auxiliary pressure device for stress urinary incontinence as defined in the appended claims, which can effectively solve the problems discussed above.

With the technical solutions of the present invention, the following technical effects can be achieved.

The present invention provides an auxiliary pressure device for stress urinary incontinence comprising an adjustment system. Through the use of the adjustment system, the present invention aims, on one hand, to improve the convenience of the expansion body entering the vagina through the imparting of the first state, thus improving the comfort during insertion into the vagina, and on the other hand, to improve the positional stability of the expansion body in the vagina to avoid discomfort caused by position changes during use, as well as embarrassment caused by accidental falling off, minimizing the doctor's intervention.

### Description of the Drawings

In order to more clearly illustrate the embodiments of the present invention or the technical solutions in the prior art, the accompanying drawings to be used in the description of the embodiments or prior art will be briefly described below. It is obvious that the accompanying drawings in the following description are only some of the embodiments recorded in the present invention, and other accompanying drawings can be obtained according to these accompanying drawings without creative work for those of ordinary skill in the art.
FIG. 1 is a schematic diagram illustrating the transition of the expansion body from a first state to a second state;
FIG. 2 is a schematic diagram of the expansion body connected to the force application part according to an embodiment;
FIG. 3 is a schematic diagram of the fitting range;
FIG. 4 is a schematic diagram of the expansion body connected to the force application part when three lead-out portions are provided, with the lead-out portion including a partially spherical fitting end;
FIG. 5 is a schematic diagram of the expansion body connected to the force application part when two lead-out portions are provided, with the lead-out portion including a partially spherical fitting end;
FIG. 6 is a schematic diagram of the expansion body in FIG. 4 rotating to its maximum position inside the vagina;
FIG. 7 is a schematic diagram of the expansion body in FIG. 5 rotating to its maximum position inside the vagina;
FIG. 8 is a schematic diagram of the expansion body connected to the force application part when two lead-out portions are provided, with the lead-out portion including a partially annular fitting end;
FIG. 9 is a schematic diagram when the deformable structure is an ellipsoidal structure;
FIG. 10 is a schematic structural diagram of the adjustment system;
FIG. 11 is a perspective view of the expansion body fully accommodated by the adjustment system in FIG. 10;
FIG. 12 is a cross-sectional view of the fitting position between the end of the outer tube body and the vaginal wall;
FIG. 13 is a schematic diagram of the expansion body partially accommodated by the adjustment system;
FIG. 14 is a cross-sectional view of the ellipsoidal expansion body fully accommodated by the adjustment system in FIG. 10;
FIG. 15 is a schematic diagram of the force analysis of the deformable structure after installation of the permanent magnets;
FIG. 16 is a schematic diagram of the opening process of the normally closed open end;
FIG. 17 is a schematic diagram of the direction of external force received by the expansion body;
FIG. 18 is an schematic exploded diagram of a set of like-pole permanent magnets relative to the expansion body;
FIG. 19 is a schematic diagram of the relative rotation of the roots of two lead-out portion;
FIG. 20 is a schematic diagram when the adjustment system is an elastic structure;
FIG. 21 is a schematic diagram of the process in which the middle part of the deformable structure obtains the first state by external force and reaches the second state through the action of the adjustment system;
FIG. 22 is a schematic diagram of the use of the positioning block;
FIG. 23 is a partially enlarged view at A in FIG. 22; and
FIG. 24 is a schematic diagram of the change process of the expansion body from the first state to the second state when the adjustment system is a pressure system.

Reference signs: 01. First state; 02. Second state; 03. Fitting range; 1. Expansion body; 11. Lead-out portion; 11a. Root; 11b. Fitting position; 11c. Distal end; 12. Groove; 2. Adjustment system; 21. Outer tube body; 21a. Open end; 21b. Annular edge; 21c. Curved outer surface; 22. Push rod; 23. Positioning block; 3. Force application part; 4. Vaginal wall; 51. First permanent magnet; 52. Second permanent magnet; 53. Third permanent magnet; 54. Fourth permanent magnet; 61. Delivery pipeline; 62. Valve body structure.

### Description of the Embodiments

The technical solutions in the embodiments of the present invention will be described clearly and completely in conjunction with the accompanying drawings in the embodiments of the present invention. Obviously, the described embodiments are only a portion of the embodiments of the present invention, rather than all the embodiments.

Unless otherwise defined, all technical and scientific terms used herein have the same meaning as commonly understood by those skilled in the art of the present invention. The terms used herein are for the purpose of describing specific embodiments only and are not intended to limit the invention. The term "and/or" as used herein includes any and all combinations of one or more of the related listed items.

An auxiliary pressure device for stress urinary incontinence comprises: an expansion body 1 with a structure for insertion into the vagina, including a first state 01 before insertion into the vagina, and a second state 02 after insertion into the vagina; an adjustment system 2 for maintaining and/or releasing the first state 01 of the expansion body 1; and a force application part 3 connected to the expansion body 1, with a traction structure for the user to apply force to move the expansion body 1 out of the vagina.

The present invention provides an auxiliary pressure device for stress urinary incontinence, comprising an adjustment system 2. Through the use of the adjustment system 2, the present invention aims, on one hand, to improve the convenience of the expansion body 1 entering the vagina through the imparting of the first state 01, thus improving the comfort during insertion into the vagina, and on the other hand, to improve the positional stability of the expansion body 1 in the vagina to avoid discomfort caused by position changes during use, as well as embarrassment caused by accidental falling off, minimizing the doctor's intervention.

After the expansion body 1 is inserted into the vagina, the force application part 3 is partially placed outside the human body. The soft material can be used to reduce the influence on the human body. Flexible threads may be a common choice and are conventionally implementation as cotton threads. Of course, this material is not the only choice for the force application part 3 according to the present invention. Other materials that are acceptable to the human body and do not cause discomfort during friction are also within the scope of protection of the present invention.

Preferably, the expansion body 1 is an integrated structure. In this way, the expansion body 1 can have better overall integrity, which on one hand ensures the stability of the structure, and on the other hand makes it easier to control the expansion body into a shape comfortable for the human body and obtains a more beautiful appearance.

Regarding the above embodiment, the adjustment system 2 may be implemented in the following specific ways, by which the technical object of the present invention can be more easily and clearly understood by those skilled in the art.

### Embodiment 1 (not part of the invention)

In this embodiment, the adjustment system 2 is configured to maintain the first state 01 of the expansion body 1. When the adjustment system 2 is separated from the expansion body 1, the expansion body 1 naturally acquires the second state 02.

Specifically, the expansion body 1 comprises at least two lead-out portions 11 evenly distributed along the circumferential direction. The respective lead-out portions 11 are connected at roots 11a and free at distal ends 11c thereof. The adjustment system 2 is configured to maintain the first state 01 of the expansion body 1. In the first state 01, the distal ends 11c are close to or fit with each other; and in the second state 02, the distal ends 11c are separated from each other. The force application part 3 is connected to the connection of the respective lead-out portions 11.

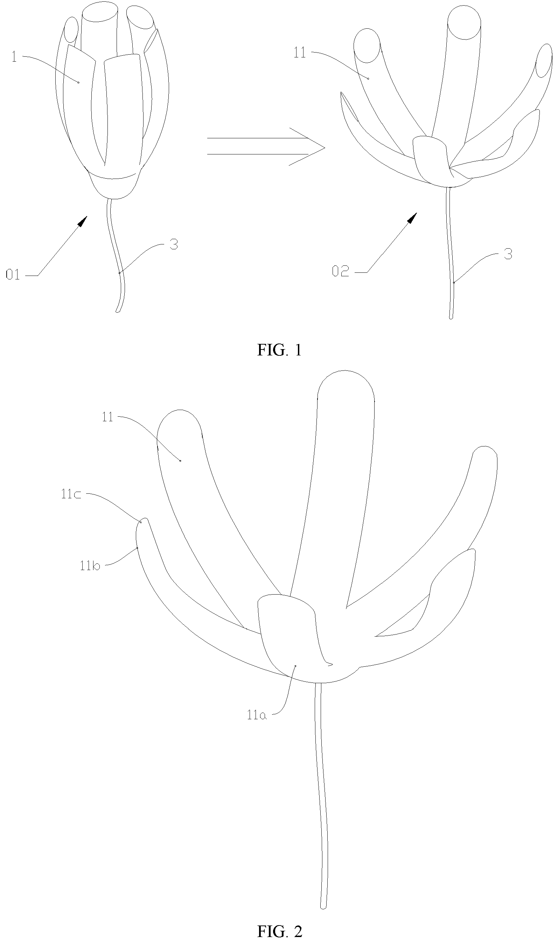

A structural form of the above embodiment is shown in FIG. 1. In the above embodiment, in the first state 01, when the distal ends 11c of the lead-out portions 11 move close to or fit with each other, it will inevitably reduce the coverage of the distal ends 11c of the respective lead-out portions 11. This reduced state is the main reason for increasing convenience in entering the vagina. In the second state 02, the distal ends 11c are separated from each other to increase the coverage and thus the fitting force with the vaginal wall 4, whereby the vaginal wall 4 is be deformed due to extrusion, increasing the extrusion force on the urethra and effectively improving the stress urinary incontinence.

In this embodiment, the expansion body 1 is preferably integrally formed. The lead-out portions 11 are deformed due to the extrusion of the adjustment system 2. After the expansion body 1 is separated from the adjustment system 2, the respective lead-out portions 11 are elastically reset and returns to the original expanded state. In this way, given that the material of the expansion body 1 is certain, it is possible to obtain different extrusion forces against the vaginal wall 4 after the expansion body is inserted into the vagina by reasonably controlling the size of the expansion body 1 in the second state 02. The expansion body 1 can thus be available in different models for choice and use by users with different needs.

In order to further improve the comfort of use, as a first preferred structural form of the above embodiment, the lead-out portion 11 is an elastically curved structure that bends inwards, and in the second state 02, the distal end 11c of the lead-out portion 11 is separated from the vaginal wall 4.

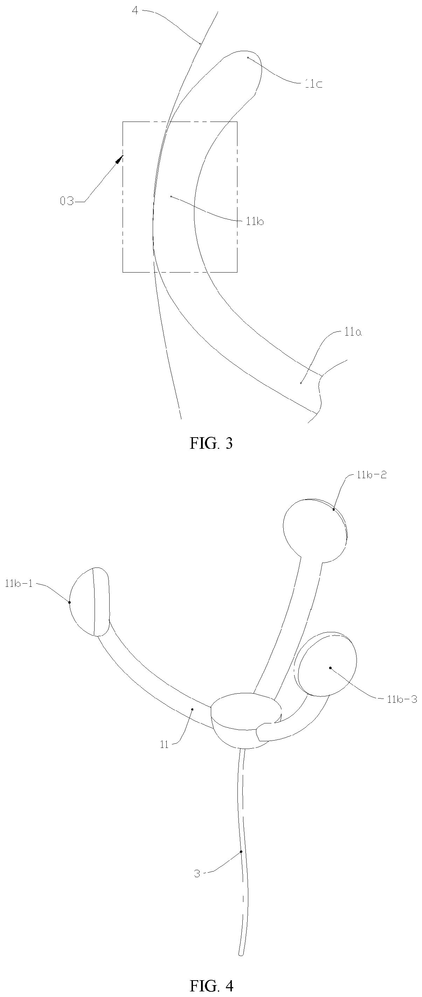

As shown in FIGS. 2 and 3, in this embodiment, the lead-out portion 11 includes a root 11a, a fitting position 11b with the vagina, and a distal end 11c. In this embodiment, the distal end 11c does not directly fit with the vaginal wall 4, but fits with the vaginal wall 4 through the portion between the distal end 11c and the root 11a. In this way, on one hand, the discomfort of the end compressing the vagina can be avoided, and on the other hand, the expansion body 1 can be made more stable through the fit of the middle part of the overall structure with the vaginal wall 4. The curved shape of the lead-out portion 11 imparts a relative extension of the fitting range 03 on both sides of the fitting position 11b, thereby obtaining a larger range of interaction with the vaginal wall 4, which is obviously beneficial to the stable fixation of the expansion body 1.

In this embodiment, the cross section of the curved structure may be circular, elliptical, etc. Alternatively, at least it is necessary to ensure that the side facing the vaginal wall 4 is smooth in order to ensure the comfort of the vaginal wall 4 when being compressed, while the shape of the other side can be selected in a wider range as it does not come into direct contact with the human body.

In this way, as shown in FIGS. 1 and 2, the lead-out portion 11 may be in the form of a rod and may obtain a greater area of fit with the vaginal wall 4 through appropriate expansion, thus improving comfort and positional stability. The above figure shows six uniformly distributed lead-out portions 11. This number is just one of the possible implementations, while other quantities such as 2, 3, 4, etc. are also within the scope of protection of the present invention. Of course, it is necessary to ensure that the selection of number adapts to the possibility of machining and application.

As a second structural form of this embodiment, the lead-out portion 11 can fit with the vaginal wall 4 at different positions. Specifically, the lead-out portion 11 comprises an elastic connection structure and a fitting end connected as the distal end 11c to one end of the connection structure. The other end of the connection structure serves as the root 11a. The fitting end expands outward relative to the connection structure, and fits with the vaginal wall in the second state 02.

In the first structural form, the curved structure is the structural form of the lead-out portion 11, the middle part of which fits with the vaginal wall 4. In this structural form, the curved structure is a part of the lead-out portion 11 and realizes fitting with the vaginal wall 4 through the fitting end connected thereto. Different from the above embodiment that ensures the installation stability through the optimization of the fitting position 11b, this embodiment focuses on ensuring the installation stability of the entire auxiliary pressure device through the optimization of the structure of the fitting end.

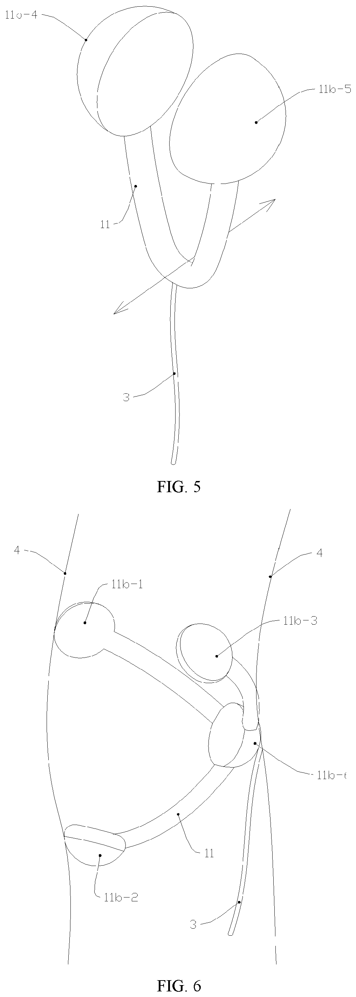

FIG. 4 shows the provision of three lead-out portions 11 correspondingly comprising three curved structures and three fitting ends. FIG. 5 shows the provision of two lead-out portions 11 correspondingly comprising two curved structures and two fitting ends. The fitting ends selected in the above figure are all partially spherical. Of course, the sphere referred to here may be a circular sphere, an ellipsoid, etc. In use, the spherical surface of the sphere is the part that fits with the vaginal wall 4. By controlling its volume, the adjustment of the area that fits with the vaginal wall 4 can be effectively achieved.

The auxiliary pressure device in the present invention can control the stability of the position after insertion into the vagina by controlling the shape and number of the fitting ends. In this embodiment, as the number of the lead-out portions 11 increases, more fitting ends may be used to fit with the inner wall of the vagina, so the size of the fitting ends can be appropriately reduced. On the contrary, when the number of lead-out portion 11 decreases, the number of fitting positions 11b with the vagina will be reduced, so the size of the fitting ends can be appropriately increased, as shown by the comparison between FIG. 4 and FIG. 5. Of course, these situations are only for considerations such as product cost and does not serve as a basis for limiting the scope of protection of the present invention.

In use, different fitting positions 11b are formed between the fitting ends and the vaginal wall 4 in this embodiment, such as the fitting positions 11b-1 to 11b-3 shown in FIG. 4, and the fitting positions 11b-4 to 11b-5 shown in FIG. 5. In use, there is an unavoidable situation, that is, the rotation of the fitting position 11b relative to the vaginal wall 4 . In the above first structural form, due to the enlargement of the fitting range 03, contact with the vaginal wall 4 can be achieved through an approximately bow-shaped structure, so that the above problem can be appropriately solved. Specifically, in the second structural form, in which the fitting ends are partially spherical, the connection of the roots 11a of the respective lead-out portions 11 may be used as an additional fitting point with the vaginal wall 4, as shown in FIG. 6, which shows the state of the expansion body 1 rotating to the extreme position on one side in the vagina. In this state, the connection of the roots 11a of the respective lead-out portions 11 may form a new fitting position 11b-6, thereby limiting further rotation. In this state, the extrusion force of the fitting position 11b-6 on the vaginal wall 4 is smaller than the extrusion force of the fitting positions 11b-1 to 11b-3 on the vaginal wall 4. Therefore, the fitting position 11b cannot play the role of positioning the auxiliary pressure device, but only achieves the purpose of preventing rotation.

When the expansion body 1 rotates, the force application part 3 will inevitably change its position. However, due to the limitation of the space in the vagina, the change in position will not affect the length outside the human body. In order to improve the comfort of use, the connection of the roots 11a of the respective lead-out portions 11 is preferably realized by a smoother structure.

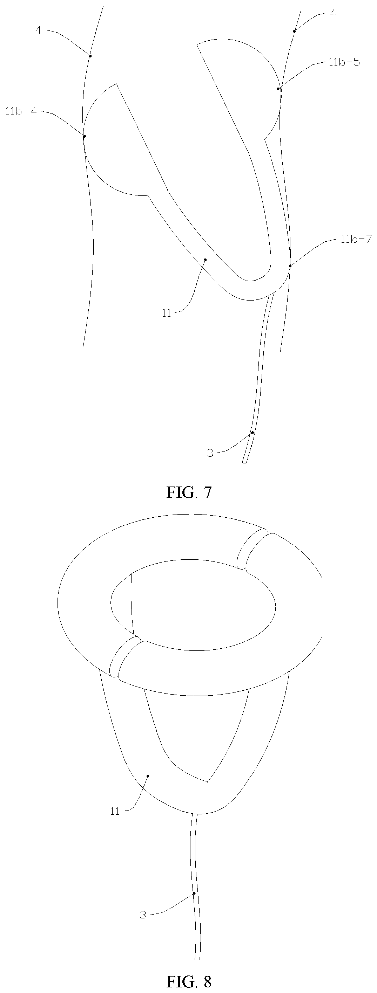

When the number of lead-out portions 11 increases relative to the case of three lead-out portions, the effect achieved is generally the with that achieved by the case with the three lead-out portions. As for the case in which only two lead-out portions 11 are provided, the effect is also the same and the only difference is that the expansion body 1 has two obviously different rotation directions: a first rotation direction, as shown in FIG 7, where the expansion body rotates in the distribution direction of the two lead-out portions 11, and a second rotation direction, as shown by the arrows in FIG 5, where the expansion body rotates relative to both sides of the distribution direction of the two lead-out portions 11. The distribution direction here is the plane direction shown in FIG. 7. Of course, regardless of the direction, the degree of rotation can be limited by the fitting position 11b-7 shown in FIG. 7.

When the fitting ends are partially annular, as shown in FIG. 8, the technical objective achieved is the same as that of partially spherical fitting ends, and the difference lies in the contact position. A larger contact area with the vaginal wall 4 in the circumferential direction can be obtained in an annular manner. Different from the bending direction of the curved structure, the bending direction of the annulus is perpendicular to the direction of insertion into the vagina, while the complete structure's bending is in compliance with the direction of insertion into the vagina.

In this embodiment, the cross section of the annulus may be circular or elliptical. Alternatively, at least it is necessary to ensure that the side facing the vaginal wall 4 is curved to ensure the comfort of the vaginal wall 4 when being pressed, while the shape of the other side can be selected in a wider range as it does not come into direct contact with the human body.

In order to limit the rotation angle through the connection of the roots 11a of the lead-out portions 11, the length of the lead-out portions 11 can be appropriately extended, so as to achieve the limit of rotation at a smaller rotation angle. Of course, the length limit may be selected based on the level of comfort that the human body can perceive.

As another preferred mode of this embodiment, the expansion body 1 is a hollow deformable structure. The adjustment system 2 is configured to maintain the first state 01 of the expansion body 1. In the first state 01, the internal cavity of the expansion body 1 is compressed and deformed, and in the second state 02, the compressed and deformed internal cavity is at least partially restored. The force application part 3 is connected to one end of the expansion body 1.

In the above embodiment, the expansion body 1 may be made of elastic and non-absorbent structure, for example, a medical-grade silicone structure, a plastic structure, or a metal structure. The material is only intended to apply pressure to the vaginal wall 4, and the pressure may be the deformation recovery force caused by bending or the deformation recovery force caused by compression deformation. Obviously, in the aforementioned arrangement of the lead-out portion 11, the elastic restoring force generated by bending is the main reason for its fixation relative to the vaginal wall 4, while in the form of the deformable structure, the elastic restoring force generated by compression deformation is the main reason for its fixation relative to the vaginal wall 4. Of course, both compression deformation and bending can occur simultaneously, and the combined elastic restoring force of the two can also ensure the stable fixation of the expanding body 1.

The deformable structure is an elastic structure that has a maximum size in the middle part along the direction of insertion into vagina and gradually shrinks and extends toward both sides, wherein the length in the extension direction is greater than the maximum width of the middle part.

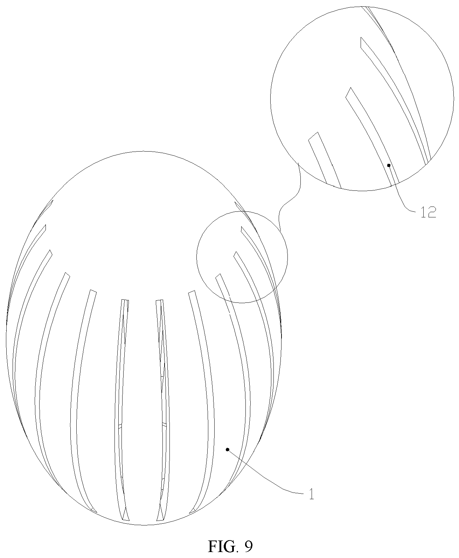

The shrinkage on both sides of the deformable structure may be symmetrical or asymmetrical, or uniform or non-uniform. The ellipsoidal shape is preferred. The long axis direction of the ellipsoid is the direction of insertion into the vagina, namely, the extension direction, as shown in FIG. 9. Therefore, a larger fitting area with the vaginal wall 4 can be obtained through a flatter sidewall around the short axis. In this embodiment, the cavity of the deformable structure is intended to accommodate the deformation of the outside when pressed and to reduce the weight of the entire expansion body 1.

The side wall of the expansion body 1 is provided with a plurality of through areas, namely, a plurality of grooves 12 distributed along the length direction of the deformable structure. By providing the grooves 12, on one hand, it will be more convenient for the expansion body 1 to achieve the first state 01 relative to the adjustment system 2, and on the other hand, it will keep the pressure inside and outside the vagina relatively constant during the process of entering the vagina, thereby avoiding the problem of difficult entry caused by local high pressure.

In the above embodiment, the expansion body 1 compresses against the vaginal wall 4 due to its own deformation or the compressibility of the material, and the extrusion force is achieved by itself. In order to further improve the extrusion effect on the vaginal wall 4, preferably, a set of unlike-pole permanent magnets are arranged on the inner wall of the expansion body 1 in the opposite directions, and the opposite directions indicate the direction of insertion into the vagina.

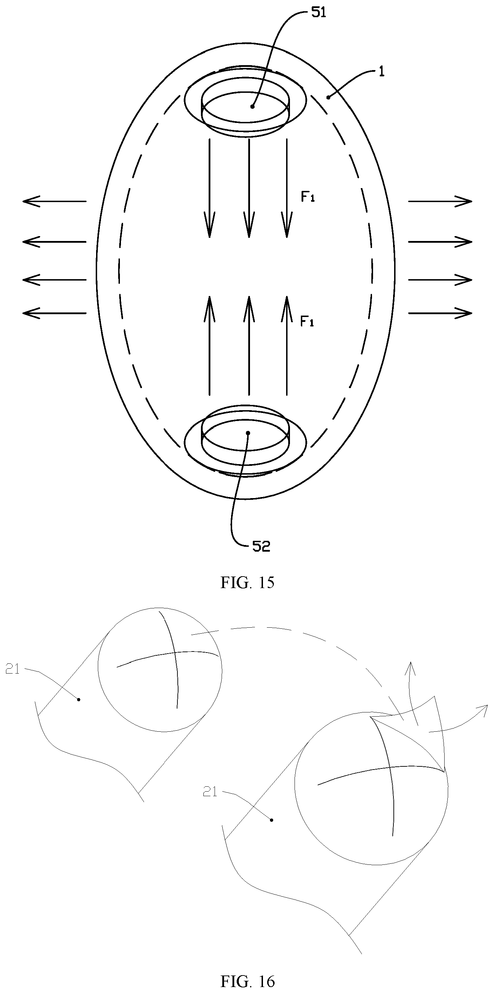

As shown in FIG. 15, a set of unlike-pole permanent magnets includes a first permanent magnet 51 and a second permanent magnet 52 that are characterized by attracting each other. The attractive force is indicated by the F1 direction in the figure. When the deformable structure is an ellipsoidal structure, arranging a set of such magnets on both sides of the long axis of the ellipsoid can cause the deformable structure to have the effect of being compressed and extending in the circumferential direction of the short axis. This effect is obviously more favorable for fixation in the vagina.

The arrangement of unlike-pole permanent magnets can be applied to a wider range of shapes. Elliptical shape is one of the comfortable structural forms. It should be noted that other structural forms that can generate a restoring force after elastic deformation and undergo deformation due to the mutual attraction of two magnets through the installation of unlike-pole permanent magnets also fall within the scope of protection of the present invention.

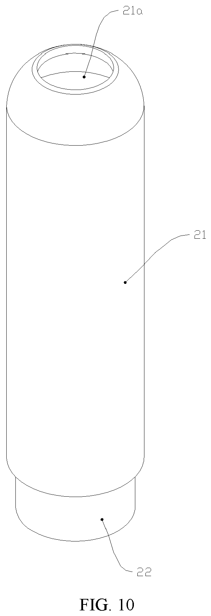

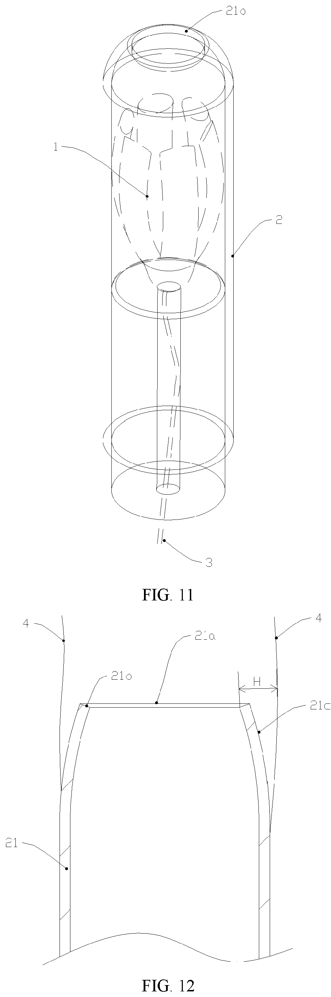

In this embodiment, as a specific implementation of the adjustment system 2 configured to maintain the first state 01 of the expansion body 1, as shown in FIGS. 10 and 11, the adjustment system 2 comprises an outer tube body 21 and a push rod 22. One end of the outer tube body 21 is an open end, at which the expansion body 1 is at least partially inserted into the outer tube body 21, and the other end of the outer tube body 21 is used for the force application part 3 to be led out. The push rod 22 is inserted from the end of the outer tube body 21 through which the force application part is led out. The expansion body 1 is moved out of the open end 21a under the action of the push rod 22.

In use, the expansion body 1 is placed into the vagina through the adjustment system 2. After the position of the end of the outer tube body 21 is determined, push the push rod 22 to push the expansion body 1 out of the open end 21a, so that the expansion body 1 is in a free state to achieve the second state 02, and then remove the adjustment system 2 from the vagina.

During the above process, the user's hands are always outside the human body, which is more hygienic. When designing the structure of the adjustment system 2, the shape of the outer tube body 21 can be optimized to provide the human body with greater comfort during the process of entering the vagina. FIG. 11 shows the situation where the expansion body 1 is entirely located inside the outer tube body 21. This situation is more suitable for the form where the lead-out portion 11 is an curved structure that bends inwards.

As a preferred embodiment, the open end 21a is provided on the curved outer surface 21c of the outer tube body 21. Therefore, the outer tube body 21 can enter the human body more gently. At the same time, the curved surface will cause the annular edge 21b of the open end 21a to retract inward relative to the vaginal wall 4 after the outer tube body 21 is inserted into the vagina, thereby forming a distance H as shown in FIG. 12 from the vaginal wall 4. The distance H prevents the expansion body 1 from coming into contact with the vaginal wall 4 during the state transition as much as possible during the removal of the expansion body 1 from the outer tube body 21. This can also further improve the comfort of use.

From the perspective of optimization of the inner wall, the inner wall of the outer tube body 21 transitions to the edge of the open end 21a through curved surface contraction. As shown in FIGS. 11 and 12, this achieves guidance during the removal of the expansion body 1 from the outer tube body 21, allowing the user to insert it into a designated position in the vagina by applying less force.

The structural form of the outer tube 21 in this embodiment serves as a guiding part and participates in the insertion process of the expansion body 1, that is, it not only maintains the first state 01 of the elastic expansion body, but also serves as a carrier. Regarding the selection of structure, the outer tube body 21 may also be an elastic and non-absorbent structure, for example, a medical-grade silicone structure, a plastic structure, or a metal structure.

The side wall of the outer tube body 21 may be a continuous or a hollowed structure. Of course, in order to ensure the comfort of use, the outer wall of the outer tube body 21 is preferably smooth. The length of the outer tube body 21 needs to ensure that after the outer tube body 21 is inserted into the human body to a set length, there is still enough length for the user to operate the push rod 22 outside the human body. Regardless of the continuous structure or the hollowed structure, they both can offer smoothness and have their own advantages. Specifically, when the side wall is in a continuous structure, as shown in FIGS. 10 and 11, it is more convenient for machining, and is also less difficult in terms of cleaning when the adjustment system 2 is a non-disposable product. When the side wall is a hollowed structure, the hollow pattern can be selected according to actual needs, but the advantage that can be achieved with various hollow patterns is that during the process of entering the vagina, the air in the vagina can better circulate through the hollowed parts, thereby maintaining the pressure in the space and avoiding resistance caused by local high pressure caused by the entry of the adjustment system 2.

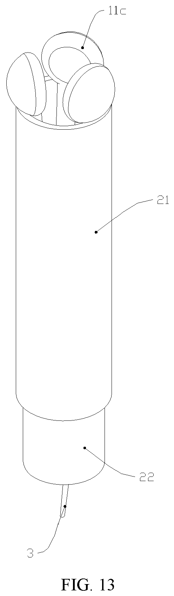

FIG. 13 shows the situation where the expansion body 1 is partially located inside the outer tube body 21. This situation is more suitable for the form where the lead-out portion 11 includes a curved structure, and a fitting end that serves as the distal end 11c and is located outside the outer tube body 21. In this case, the fitting end will inevitably come into contact with the vaginal wall 4 during the process of entering the vagina, thereby causing the vaginal wall 4 to expand appropriately for insertion. When the expansion body 1 is in place and pushed by the push rod 22, as the roots 11a are moved out, the respective fitting positions 11b gradually achieve effective support for the vaginal wall 4, and then the adjustment system 2 can be removed. In this way, the size of the adjustment system 2 can be reduced, and the discomfort during insertion can also be alleviated to a certain extent.

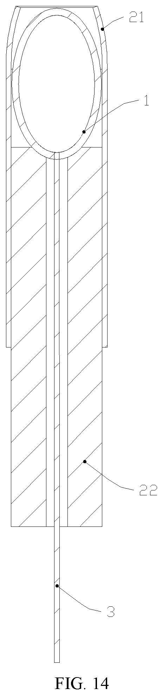

FIG. 14 shows the installation of the expansion body 1 of the deformable structure relative to the adjustment system 2. The specific use is the same as that of the structure shown in FIGS. 11 and 13 and will not be repeated here.

As for the improvement of the outer tube body 21 and the push rod 22, the inner wall of the outer tube body 21 linearly guides the push rod 22, which is not limited by the shape of the outer wall of the outer tube body 21. The linear guidance is the simplest way for the user to execute the pushing action, simply by making the outer wall of the pushing rod 22 fit with the inner wall of the outer tube body 21 and ensuring that the fitting positions 11b can extend in a linear direction. In order to facilitate the operation of the force application part 3, the push rod 22 is a hollow structure with a hollow portion through which the force application part 3 passes.

Regarding the open end 21a, the present invention provides the following two implementations. According to a first implementation, the open end 21a is in a normally open state, and according to a second implementation, the outer tube body 21 is an elastic body and the open end 21a is in a normally closed state and is opened under the action of external extrusion force.

FIGS. 10 to 14 show the open end 21a in a normally open state. To ensure hygiene before use, an open end 21a that is normally closed and opened under set conditions can also be provided, as shown in FIG 16, which shows a relatively simple open end 21a in a normally closed state. By providing a through trace in the complete structure, the passage of the expansion body 1 can be achieved through the opening of the through trace.

The crossing through traces are a conventional approach, as shown in Figure 16, which involves crossing two through traces. Of course, more through traces intersecting forms are also within the scope of protection of the present invention. Of course, in this case, it is necessary that the outer tube body 21 is formed of an elastic material. The through traces may be obtained by cutting, whereby the normally closed state can be achieved by fitting both sides of the traces. The opening process can be achieved simply by deformation of the through traces. The direction of the arrow in FIG. 16 is the direction of deformation.

### Embodiment 2

The above Embodiment 1 illustrates the case where the adjustment system 2 is configured to maintain the first state 01 of the expansion body 1. When the adjustment system 2 is separated from the expansion body 1, the expansion body 1 naturally acquires the second state 02. The difference in this embodiment is that the adjustment system 2 is configured to release the first state 01 of the expansion body 1, while the maintenance of the first state 01 is achieved through the application of force by the user's hand, which is the external force hereinafter.

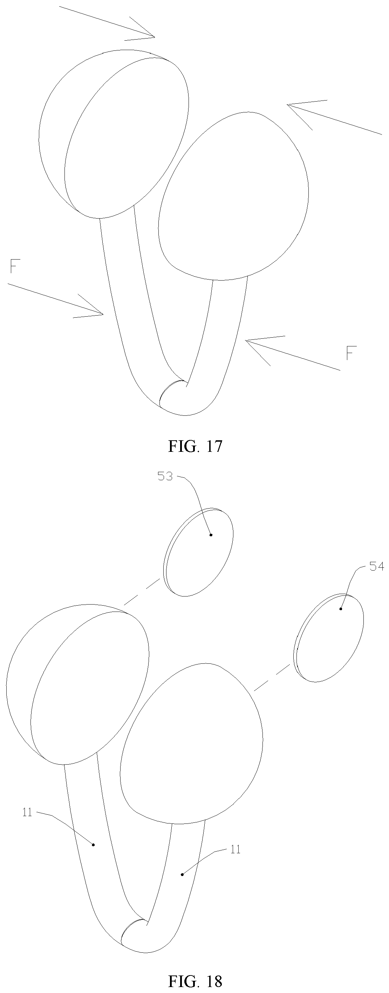

This embodiment provides an auxiliary pressure device that requires the user's hand to reach into the vagina. As shown in FIG. 17, the arrow direction of force F represents the direction of force applied by the hand towards the lead-out portions 11, thereby bringing the respective distal ends 11c closer and obtaining a state in favor for entering the vagina.

The expansion body 1 comprises at least two lead-out portions 11 evenly distributed along the circumferential direction. The respective lead-out portions 11 are connected at roots 11a and free at distal ends 11c thereof. The adjustment system 2 is configured to release the first state 01 of the expansion body 1 to reach the second state 02. In the first state, the distal ends 11c move close to or fit with each other under the action of external force, and in the second state 02, the external force is released and the respective distal ends 11c are separated from each other under the action of the adjustment system 2. The force application part 3 is connected to the connection of the respective lead-out portions 11.

In this case, when the force F is released, the distal ends 11c of the respective lead-out portions 11 move away from each other due to the adjustment system 2, wherein the lead-out portions 11 are elastic, and/or the connection of the respective lead-out portions 11 is elastic. With the elasticity of the above structures, the lead-out portions 11 themselves can undergo elastic deformation, and/or the connection of the respective lead-out portions 11 can undergo elastic deformation under the action of the external force, so that after the user removes the external force, the recovery of the deformation will generate a restoring force. The restoring force here is beneficial for the fixation of the entire expanding body 1 relative to the vaginal wall 4. However, in this embodiment, the above-mentioned restoring force is not the main factor in fixing the expansion body 1 relative to the vaginal wall 4, but only plays an auxiliary role.

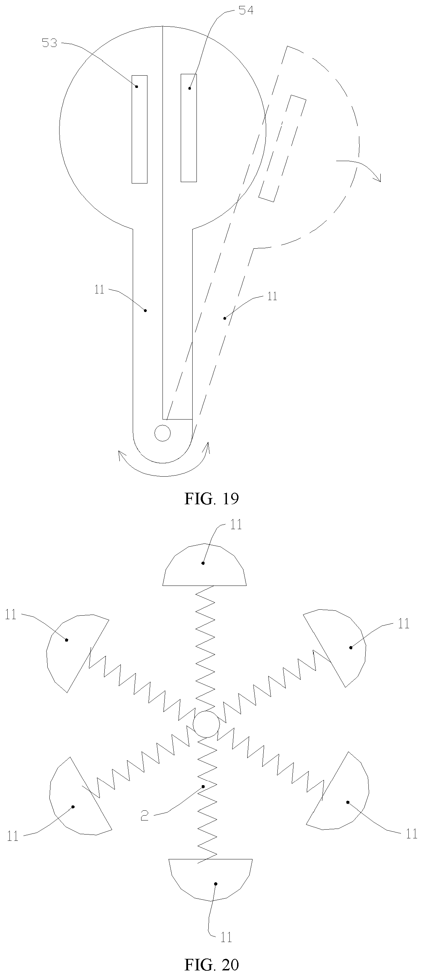

In the above case, or in the case where the above elasticity is not provided, as another preferred embodiment, the roots 11a of the respective lead-out portions 11 are arranged to rotate relative to each other. As shown in FIG. 19, this can also ensure that the respective lead-out portions 11 reach the second state 02 after relative rotation. The rotational arrangement allows the variation in relative position of the respective lead-out portions 11.

The relative rotational arrangement here may be a rotational connection between two lead-out portions 11 as shown in FIG. 19, or a rotational connection between all lead-out portions 11 with an additional third-party structure, so that the relative rotation is achieved through rotation relative to the third party.

This embodiment provides the following implementation of the adjustment system 2.

According to a first implementation, the adjustment system 2 is a set of like-pole permanent magnets arranged in one-to-one correspondence with the distal ends 11c and fixedly arranged relative to the distal ends 11c. The like-pole permanent magnets may be respectively embedded in the distal ends 11c of the respective lead-out portions 11.

This ensures that no discomfort to the human body will be caused during use, because the material of the expansion body 1 itself in the present invention does not affect the magnetism. As shown in FIG. 18, the third permanent magnet 53 and the fourth permanent magnet 54 are the permanent magnets that need to be embedded.

During the machining process, it is preferred that the permanent magnets are seamlessly embedded, so that the outer surface of the expansion body 1 can be considered as an integrated structure. This not only ensures the safety of use, but also effectively improves the aesthetics of the product, thereby bringing better user experience.

In order to ensure sufficient magnetism, the size of the permanent magnet needs to be guaranteed, and in order to ensure sufficient installation position, the embedding position of the permanent magnet can be appropriately increased. The fitting end in the above Embodiment 1 can achieve this goal, and of course, it can also increase the fitting area with the vaginal wall 4.

The optimization scheme for increasing the fitting area in the above Embodiment 1 is also applicable to this embodiment, so it will not be repeated here.

According to a second implementation, the adjustment system 2 is an elastic structure connected to the respective lead-out portions 11 and can be deformed under the extrusion of any of the lead-out portions 11. The schematic diagram shown in FIG. 20 shows a way for the adjustment system 2 to achieve elasticity, in which the adjustment system is connected to the respective lead-out portions 11 through an elastic protrusion position in one-to-one correspondence with the respective lead-out portions 11. It should be noted that the line structure in the figure only represents an elastically deformable structure, and it can be in any form such as a spring structure, a rubber structure, or a compressible airbag. The respective lead-out portions 11 fit with the vaginal wall 4, so it is necessary to ensure that there is no difference in the deformation of the elastic structure in the direction towards each of the lead-out portions 11, thereby ensuring the equivalence of use in all directions.

When the user's hand exerts force on the lead-out portions 11, the elastic structure will deform to allow the respective lead-out portions 11 to move close each other to achieve the first state 01. When the external force is removed, the reset of elastic deformation can act on the expansion body 1 so that the expansion body reaches the second state 02.

According to a third implementation of the adjustment system 2, the expansion body 1 is a hollow deformable structure. The adjustment system 2 is configured to release the first state 01 of the expansion body 1 to reach the second state 02. In the first state 01, the deformable structure causes the internal cavity of the expansion body 1 to be compressed and deformed under the action of external force, and in the second state 02, the external force is released and the internal cavity is at least partially restored under the action of the adjustment system 2. The force application part 3 is connected to one end of the expansion body 1.

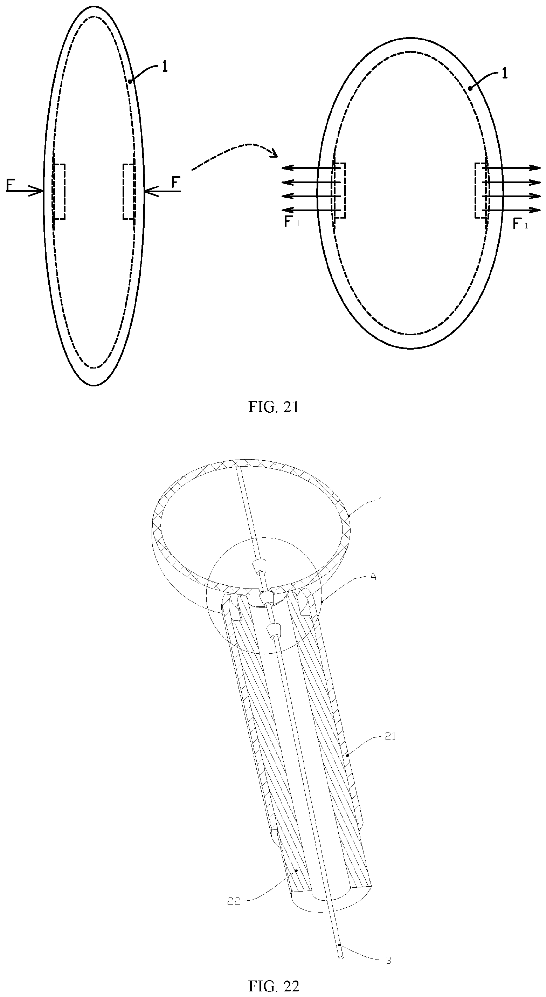

FIG. 21 shows the deformable structure of the expansion body 1. Similarly, the contraction on both sides of the deformable structure can be symmetric or asymmetric, as well as uniform or non-uniform. The ellipsoidal shape is preferred, and the advantages of the ellipsoidal shape are described in the above Embodiment 1, which will not be repeated here.

As a specific optimization, the deformable structure is an elastic structure that has a maximum size in the middle part along the direction of insertion into vagina and gradually shrinks and extends toward both sides, wherein the length in the extension direction is greater than the maximum width of the middle part, the inner wall of the expansion body is provided with a plurality of like-pole permanent magnets evenly distributed around the middle part of the deformable structure, and the external force acts on the outer wall of the middle part.

Referring also to FIG. 21, F in the figure represents the direction of the user's external force, and F1 in the figure represents the mutual repulsive force between the permanent magnets, so that there is sufficient fitting force between the expansion body 1 and the vaginal wall 4.

In this embodiment, the expansion body 1 may have an elastic structure, so that it can also produce an extrusion effect on the vaginal wall 4 by a certain restoring force through elastic reset. Similarly, in this embodiment, the above-mentioned restoring force is not the main factor in fixing the expansion body 1 relative to the vaginal wall 4, but only plays an auxiliary role.

As described in the above embodiment, the elastic structure of an ellipsoidal structure is preferred. Of course, drum shaped or other profiled structures that meet the above conditions are also within the scope of protection of the present invention. Taking the ellipsoid as an example, the long axis direction of the ellipsoid is the direction of insertion into the vagina. Considering that the ellipsoid has a long axis and a short axis and the direction of action of the like-pole permanent magnets, even if a certain deflection occurs during the insertion into the vagina, the set directions of the long and short axes will be naturally adjusted in the vagina, thereby achieving the expected extrusion effect on the vaginal wall 4.

According to a fourth implementation of the adjustment system 2, the deformable structure is an elastic structure that has a maximum size in the middle part along the direction of insertion into vagina and gradually shrinks and extends toward both sides, wherein the length in the extension direction is greater than the maximum width of the middle part. One end of the force application part 3 is connected to the inner wall of the internal cavity at one end in the length direction of the deformable structure, and the other end of the force application part 3 passes through the other end of the internal cavity. The adjustment system 2 comprises a positioning block 23 fixed on the force application part 3. The positioning block 23 includes a ready-to-use state in which it is located in the internal cavity, and a positioning state in which it is located outside the internal cavity and maintains press fit with the outer wall of the expansion body. The transition from the ready-to-use state to the positioning state is achieved in the process of maintaining the position of the expansion body 1 in the vagina by external force and pulling the force application part 3 by external force.

In this embodiment, the above optimization scheme is implemented for the purpose that the adjustment system 2 is configured to release the first state 01 of expansion body 1. Specifically, when the expansion body 1 is in place, the force application part 3 is pulled to exert force on the connection between the expansion body 1 and the internal cavity, so that the expansion body 1 undergoes elastic deformation. The deformation process occurs in the direction perpendicular to the force exerted by the force application part 3 on the expansion body 1, which is the direction of extrusion on the vaginal wall 4. By controlling the position of the positioning block 23, the final degree of deformation can be controlled, thereby achieving control over the extrusion force on the vaginal wall 4.

In order to ensure that the positioning block 23 can fit with the outer wall of the expansion body 1 outside the internal cavity, the elastic structure of the deformable structure can be utilized. When passing through the penetration position, the positioning block compresses the elastic structure to deform the penetration position, thereby passing through the penetration position, and the deformation recovers after the positioning block passes, so that the positioning block 23 can be blocked outside the elastic structure.

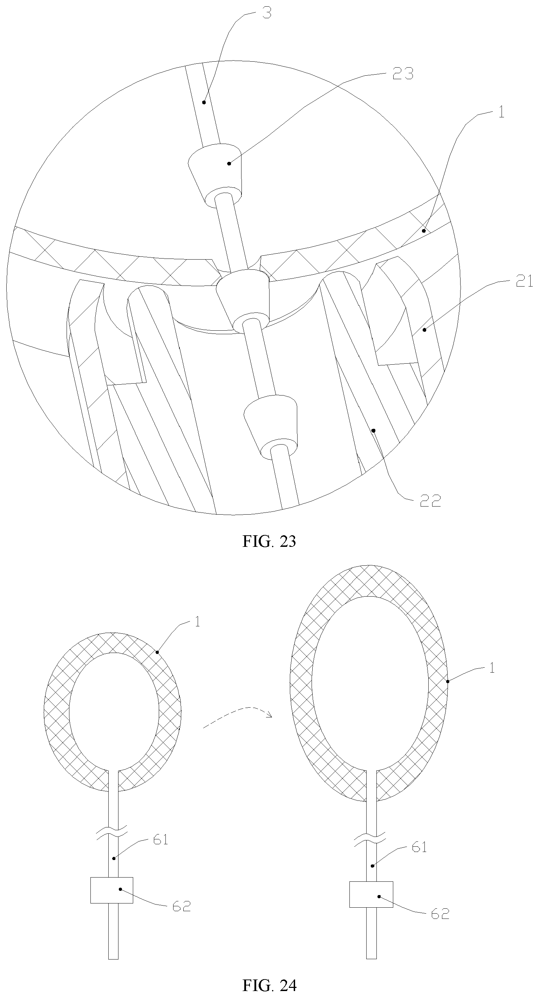

For better blocking effect, the exterior of the positioning block 23 gradually shrinks in the direction of removal from the internal cavity. The frustum shape shown in FIG. 23 is an implementation of the present invention. Correspondingly, the hole on the expansion body 1 for the positioning block 23 to move out can adopt the same shrinking form and direction as the positioning block 23, thereby achieving guidance on one hand, and ensuring stable positioning of the exterior of the positioning block 23 on the other hand.

The positioning block 23 is located on the force application part 3 and is provided in a number of at least two side by side at an interval along the direction of removal from the internal cavity. Therefore, the adjustment of different extrusion forces on the vaginal wall 4 can be achieved by fitting different positioning blocks 23 to the outer wall of the expansion body 1.

### Embodiment 3

The difference between this embodiment and the above embodiments is that the adjustment system 2 does not separately maintain or release the first state 01, but simultaneously achieves the above two technical objectives. specifically, the expansion body 1 is a hollow deformable structure. The adjustment system 2 is configured to maintain and release the first state 01 of the expansion body 1. In the first state 01, the internal cavity of the expansion body 1 is compressed and deformed, and in the second state 02, the compressed and deformed internal cavity is at least partially restored. The force application part 3 is connected to one end of the expansion body 1.

In this embodiment, the deformable structure is an elastic structure that has a maximum size in the middle part along the direction of insertion into vagina and gradually shrinks and extends toward both sides, wherein the length in the extension direction is greater than the maximum width of the middle part. One end of the force application part 3 is connected to the inner wall of the internal cavity at one end of the long axis of the ellipsoid, and the other end of the force application part 3 passes through the internal cavity at the other end of the long axis. The adjustment system 2 comprises an outer tube body 21 and a push rod 22. One end of the outer tube body 21 is an open end, at which the expansion body 1 is at least partially inserted into the outer tube body 21, and the other end of the outer tube body 21 is used for the force application part 3 to be led out. The push rod 22 is inserted from the end of the outer tube body 21 through which the force application part is led out. The expansion body 1 is moved out of the open end 21a under the action of the push rod 22. The adjustment system 2 further comprises a positioning block 23 fixed on the force application part 3. The positioning block 23 includes a ready-to-use state in which it is located in the internal cavity, and a positioning state in which it is located outside the internal cavity and maintains press fit with the outer wall of the expansion body 1. The transition from the ready-to-use state to the positioning state is achieved in the process of maintaining the position of the outer tube body 21 and/or push rod 22 and the fitting state with the expansion body 1 by external force and pulling the force application part 3 by external force.

As shown in FIGS. 22 and 23, the outer tube body 21 and the push rod 22 in this embodiment have the same function as in Embodiment 1 and can also be optimized in the same way. The positioning block 23 has the same function as in Embodiment 2 and can also be optimized in the same way. The optimization in Embodiment 1 will not be repeated. The difference is that the outer tube also needs to maintain the position of the expanding body 1 when it is inserted into the vagina and the expansion body 1 is placed externally relative to the outer tube body 21.

### Embodiment 4

Compared with Embodiment 3, this embodiment adopts a different adjustment system 2. Specifically, the adjustment system 2 is a pressure system using gas or liquid as a medium, comprising a delivery pipeline 61 and a valve body structure 62. The delivery pipeline 61 connects the internal cavity and external space of the expansion body 1. The valve body structure 62 is mounted on a portion of the delivery pipeline 61 located in the external space and is configured to open and close the internal channel of the delivery pipeline 61.

During the implementation process, the injection and extraction of the medium into and from the delivery pipeline 61 can be achieved through an external syringe or the like. The injection of the medium is performed after the expansion body 1 in the first state 01 enters the vagina. It is necessary to ensure that the delivery pipeline 61 is partially located outside the vagina, and the extrusion force on the vaginal wall 4 is adjusted by adjusting the injection amount of the medium. The valve body configuration 62 may be selected to be unidirectional to ensure entry of the medium, and to ensure the medium pressure at the time of insertion when the auxiliary pressure device is removed from the vagina. Alternatively, the valve body structure 62 may be bidirectional so that the medium can be extracted after use and then the auxiliary pressure device is removed from the vagina.

The valve body structure 62 may be located close to the expansion body 1. As the expansion body 1 is inserted into the vagina, the vagina is partially expanded by the expansion body 1 to accommodate the location of the valve body structure 62, thereby avoiding discomfort outside the human body.

The outer surface of the deformable structure in the above embodiments of the present invention may be equipped with appropriate anti-slip structures to increase the friction with the vaginal wall 4, thereby ensuring positional stability. Of course, a raised surface is preferred, and of course, the amplitude of the raise needs to be effectively controlled to avoid discomfort.

Of course, in this embodiment, the outer tube body 21 and the push rod 22, which are also part of the adjustment system 2, can also be used to facilitate the insertion of the expansion body. Similarly, they can be optimized in the same way as in Embodiment 1.

The basic principles, main features and advantages of the present invention have been shown and described above. Those skilled in the industry should understand that the present invention is not limited by the foregoing embodiments. The foregoing embodiments and descriptions only illustrate the principles of the present invention. The scope of protection claimed by the present invention is defined by the appended claims.

## Claims

1. An auxiliary pressure device for stress urinary incontinence, comprising:
an expansion body (1) with a structure for insertion into the vagina, including a first state (01) before insertion into the vagina, and a second state (02) after insertion into the vagina; an adjustment system (2) for releasing the first state (01) of the expansion body (1) to reach the second state of the expansion body; and a force application part (3) connected to the expansion body (1), with a traction structure for the user to apply force to move the expansion body (1) out of the vagina;
wherein the expansion body (1) comprises at least two lead-out portions (11) evenly distributed along the circumferential direction, the respective lead-out portions (11) being connected at roots (11a) and free at distal ends (11c) thereof, wherein the adjustment system (2) is configured to release the first state (01) of the expansion body (1) to reach the second state (02), wherein in the first state (01), the distal ends (11c) are close to or fit with each other under the action of external force, and wherein in the second state (02), the external force is released and the respective distal ends (11c) are separated from each other under the action of the adjustment system (2);
wherein the force application part (3) is connected to the connection of the roots (11a) of the respective lead-out portions (11);
wherein the expansion body (1) is an integral structure, the connection of the roots (11a) of the respective lead-out portions (11) is capable of acting as an additional fitting point with the vaginal wall (4), and the connection of the roots (11a) of the respective lead-out portions (11) is realized through a smooth structure;
wherein the connection of the roots (11a) of the respective lead-out portions (11) forms a new fitting position (11b) in a state in which the expansion body (1) rotates to an extreme position on one side in the vagina, thereby limiting further rotation, and in which the extrusion force of the fitting position (11b) formed by the connection of the roots (11a) of the respective lead-out portions (11) on the vaginal wall (4) is smaller than the extrusion force of the fitting positions (11b) formed by the distal ends (11c) on the vaginal wall (4);
**characterized in that**
the adjustment system (2) is a set of like-pole permanent magnets arranged in one-to-one correspondence with the distal ends (11c) and fixedly arranged relative to the distal ends (11c);
wherein the like-pole permanent magnets are respectively embedded in the distal ends (11c) of the respective lead-out portions (11).

2. The auxiliary pressure device for stress urinary incontinence according to claim 1, wherein the roots (11a) of the respective lead-out portions (11) are arranged to rotate relative to each other.

3. The auxiliary pressure device for stress urinary incontinence according to claim 1, wherein the lead-out portions (11) are elastic, and/or the connection of the roots (11a) of the respective lead-out portions (11) is elastic.

## Patentansprüche

1. Ein Hilfsdruckgerät für Belastungsinkontinenz, bestehend aus:
einem Expansionskörper (1) mit einer Struktur zum Einführen in die Vagina, einschließlich eines ersten Zustands (01) vor dem Einführen in die Vagina und eines zweiten Zustands (02) nach dem Einführen in die Vagina; einem Verstellsystem (2) zum Lösen des ersten Zustands (01) des Expansionskörpers (1), um den zweiten Zustand des Expansionskörpers zu erreichen; und einem mit dem Expansionskörper (1) verbundenen Kraftanwendungsteil (3) mit einer Zugstruktur, mit der der Benutzer Kraft anwenden kann, um den Expansionskörper (1) aus der Vagina zu bewegen;
wobei der Expansionskörper (1) mindestens zwei gleichmäßig entlang der Umfangsrichtung verteilte Auslaufabschnitte (11) umfasst, wobei die jeweiligen Auslaufabschnitte (11) an ihren Wurzeln (11a) verbunden und an ihren distalen Enden (11c) frei sind, wobei das Verstellsystem (2) dazu konfiguriert ist, den ersten Zustand (01) des Expansionskörpers (1) aufzuheben, um den zweiten Zustand (02) zu erreichen, wobei im ersten Zustand (01) die distalen Enden (11c) unter der Einwirkung einer äußeren Kraft nahe beieinander liegen oder aneinander passen, und wobei im zweiten Zustand (02) die äußere Kraft aufgehoben wird und die jeweiligen distalen Enden (11c) unter der Einwirkung des Verstellsystems (2) voneinander getrennt werden;
wobei das Kraftanwendungsteil (3) mit der Verbindung der Wurzeln (11a) der jeweiligen Herausführungsabschnitte (11) verbunden ist;
wobei der Expansionskörper (1) eine integrale Struktur ist, die Verbindung der Wurzeln (11a) der jeweiligen Auslaufabschnitte (11) als zusätzlicher Passpunkt mit der Vaginalwand (4) dienen kann und die Verbindung der Wurzeln (11a) der jeweiligen Auslaufabschnitte (11) durch eine glatte Struktur realisiert ist;
wobei die Verbindung der Wurzeln (11a) der jeweiligen Auslaufabschnitte (11) eine neue Passposition (11b) in einem Zustand bildet, in dem der Expansionskörper (1) in der Vagina bis zu einer äußersten Position auf einer Seite rotiert und dadurch eine weitere Rotation begrenzt, und in dem die Extrusionskraft der Passposition (11b), die durch die Verbindung der Wurzeln (11a) der jeweiligen Auslaufabschnitte (11) an der Vaginalwand (4) gebildet wird, kleiner ist als die Extrusionskraft der Passpositionen (11b), die durch die distalen Enden (11c) an der Vaginalwand (4) gebildet werden;
**dadurch gekennzeichnet, dass**
das Verstellsystem (2) ein Satz von Permanentmagneten mit gleicher Polung ist, die in einer Eins-zu-eins-Entsprechung zu den distalen Enden (11c) angeordnet sind und relativ zu den distalen Enden (11c) fest angeordnet sind;
wobei die Permanentmagnete mit gleicher Polung jeweils in die distalen Enden (11c) der jeweiligen Herausführungsabschnitte (11) eingebettet sind.

2. Das Hilfsdruckgerät für Belastungsinkontinenz nach Anspruch 1, wobei die Wurzeln (11a) der jeweiligen Auslaufabschnitte (11) so angeordnet sind, dass sie relativ zueinander rotieren.

3. Das Hilfsdruckgerät für Belastungsinkontinenz nach Anspruch 1, wobei die Auslaufabschnitte (11) elastisch sind und/oder die Verbindung der Wurzeln (11a) der jeweiligen Auslaufabschnitte (11) elastisch ist.

## Revendications

1. Dispositif de pression auxiliaire pour l'incontinence urinaire d'effort, comprenant :
un corps d'expansion (1) avec une structure d'insertion dans le vagin, comprenant un premier état (01) avant l'insertion dans le vagin, et un deuxième état (02) après l'insertion dans le vagin ; un système de réglage (2) pour libérer le premier état (01) du corps d'expansion (1) pour atteindre le deuxième état du corps d'expansion ; et une partie d'application de force (3) reliée au corps d'expansion (1), avec une structure de traction permettant à l'utilisateur d'appliquer une force pour déplacer le corps d'expansion (1) hors du vagin ;
dans lequel le corps d'expansion (1) comprend au moins deux parties de sortie (11) uniformément réparties le long de la direction circonférentielle, les parties de sortie respectives (11) étant reliées aux racines (11a) et libres aux extrémités distales (11c) de celle-ci, le système de réglage (2) étant configuré pour libérer le premier état (01) du corps de dilatation (1) pour atteindre le deuxième état (02), dans lequel dans le premier état (01), les extrémités distales (11c) sont proches ou s'emboîtent les unes aux autres sous l'action de la force externe, et dans lequel dans le deuxième état (02), la force externe est libérée et les extrémités distales respectives (11c) sont séparées les unes des autres sous l'action du système de réglage (2) ;
dans lequel la partie d'application de la force (3) est reliée à la connexion des racines (11a) des parties d'entrée respectives (11) ;
dans lequel le corps d'expansion (1) est une structure intégrale, la connexion des racines (11a) des parties de sortie (11) respectives est capable d'agir comme un point d'ajustement supplémentaire avec la paroi vaginale (4), et la connexion des racines (11a) des parties de sortie (11) respectives est réalisée par une structure lisse ;
dans lequel la connexion des racines (11a) des parties de sortie respectives (11) forme une nouvelle position d'ajustement (11b) dans un état dans lequel le corps d'expansion (1) tourne jusqu'à une position extrême d'un côté dans le vagin, limitant ainsi la rotation ultérieure, et dans lequel la force d'extrusion de la position d'ajustement (11b) formée par la connexion des racines (11a) des parties d'entrée respectives (11) sur la paroi vaginale (4) est plus petite que la force d'extrusion des positions d'ajustement (11b) formées par les extrémités distales (11c) sur la paroi vaginale (4) ;
**caractérisé en ce que**
le système de réglage (2) est un ensemble d'aimants permanents aux pôles similaires disposés en correspondance biunivoque avec les extrémités distales (11c) et disposés de manière fixe par rapport aux extrémités distales (11c) ;
dans lequel les aimants permanents aux pôles similaires sont respectivement intégrés dans les extrémités distales (11c) des parties de sortie respectives (11).

2. Dispositif de pression auxiliaire pour l'incontinence urinaire d'effort selon la revendication 1, dans lequel les racines (11a) des parties de sortie respectives (11) sont disposées de manière à tourner les unes par rapport aux autres.

3. Dispositif de pression auxiliaire pour l'incontinence urinaire d'effort selon la revendication 1, dans lequel les parties d'entrée (11) sont élastiques et/ou la connexion des racines (11a) des parties d'entrée (11) respectives est élastique.
